# EUROPEAN PATENT APPLICATION

(11) **EP 4 154 887 A1**
(43) Date of publication of application: **29.03.2023**
(21) Application number: 20937996.5
(22) Date of filing: 30.06.2020
(51) Int. Cl.: A61K 31/4965, A61K 9/14, A61K 9/20, A61K 9/48, A61P 31/14

(54) **METHOD FOR TREATING RNA VIRAL INFECTIONS, INTER ALIA COVID-19 (SARS-COV-2)**

(30) Priority: 23.05.2020 RU 2020117666
(71) Applicant: Limited Liability Company "Cromis" (LLC "Cromis"), Moscow, 121205 (RU)
(72) Inventor: IVASHCHENKO, Andrey Alexandrovich, Moscow, 127576 (RU); SAVCHUK, Nikolay Filippovich, Rancho Santa Fe, alifornia 92067 (US); IVACHCHENKO, Alena Alexandrovna, Hallandale, Florida 33009 (US); IVASCHENKO, Alexandre Vasilievich, Hallandale, Florida 33009 (US); KRAVCHENKO, Dmitrii Vladimirovich, Moskovskaia oblast, Khimki, 141402 (RU); SITDEKOV, Tagir Alievich, Moscow, 109147 (RU)
(74) Representative: Zellentin & Partner mbB Patentanwälte
(86) International application number: PCT/RU2020/000321
(87) International publication number: WO 2021/242134

(57) **Abstract**

This invention relates to a novel method for treating RNA viral infections including COVID-19 (SARS-CoV-2).

A method for treating RNA viral infections, including COVID-19 (SARS-CoV-2), which consists in administering to the patient a pharmaceutical composition in the form of a tablet, capsule, pill, or powder containing Favipiravir as the active ingredient at a dose of ≥40 mg/kg/day on day 1 and at a dose of ≥16 mg/kg/day on subsequent days until the virus leaves the body, optionally in combination with concomitant medications.

## Description

This invention relates to a novel method for treating RNA viral infections, including COVID-19 (SARS-CoV-2).

There is a known method for the treatment of RNA viral infections, including the highly pathogenic A H5N1 strain of avian influenza [R.W. Sidwell at al. Antimicrob. Agents Chemother. 2007, 51(3): 845-851], using the **Favipiravir (FVP)** drug (also known as T-705 and Avigan) and patented in 1999 by the Japanese company Toyama Chemical Co [RU 2224520]. FVP shows antiviral activity against many other RNA viruses, such as arenaviruses, bunyaviruses6 and filoviruses, which are known to cause fatal Ebola hemorrhagic fever [Y. Furuta et al. Review Favipiravir (T-705), a broad spectrum inhibitor of viral RNA polymerase. Proc. Jpn. Acad., Ser.B 93, 2017, 449-463. L. Oestereich et al. Ebola virus infection with T-705 (favipiravir) in a small animal model. Antiviral Research 2014, 105, 17-21.]

FVP, sold in Japan under the brand name Avigan (Avigan Tablet 200 mg, protected by the Russian patent RU 2527766, wherein the FVP content in the tablet was claimed to be 50-95%) and in China under the brand name Favilavir (Favilavir Tablet 200 mg), is an antiviral drug used in Japan for treating influenza. Developed and now produced by Toyama Chemical Co (FujiFilm Group), it was approved for medical use in Japan in 2014 [Shiraki K., Daikoku T. Favipiravir, an anti-influenza drug against life-threatening RNA virus infections. Pharmacology & Therapeutics 2020, 107512. doi:10.1016/j.pharmthera.2020.107512].

A sudden outbreak of the new coronavirus (later named SARS-CoV-2) in Wuhan, China, in 2019, which quickly turned into a global pandemic, marked the third introduction of a virulent coronavirus into human society, affecting not only the health system, but also the global economy. Effective approaches to vaccination, prevention, and treatment of SARS-CoV-2 (COVID-19) and epidemiological control are still lacking.

In this regard, an intensive worldwide search is underway for vaccines and therapeutic agents to prevent and treat RNA viral infections, including SARS-CoV-2 (COVID-19). One practical approach as a rapid response to an emerging pandemic is to repurpose existing therapeutic agents previously intended for other viral infections, since most of these agents have already been tested for their safety.

A common method to treat RNA viral infections is oral administration of a pharmaceutical composition in the form of tablets, capsules, powders, etc. containing FVP as the active ingredient, with daily FVP doses varying widely. For example, to treat influenza, FVP was used at a dose of 1800 mg twice a day on the first day, and then 800 mg twice a day for 2-5 days [https://www.medrxiv.org/content/medrxiv/early/2020/04/08/2020.03.17.20037432. full.pdf], while to treat Ebola virus infection, FVP was given in doses of 2400 mg, 2400 mg, or 1200 mg every 8 hours on the first day and a maintenance dose of 1200 mg was given twice a day.
[https://www.thelancet.com/journals/laninf/article/PIIS1473-3099(14)71047-3/fulltext].

To treat SARS-CoV-2 (COVID-19) infections, FVP was used as follows:
- at a dose of 1600 mg twice a day on the first day, and then 600 mg twice a day for 14 days [https://www.jwatch.org/na51293/2020/04/09/favipiravir-potential-antiviral-covid-19. clinicaltrials.gov/ct2/show/NCT04349241; https://www.medicalnewstoday.com/articles/anti-flu-drug-effective-in-treating-covid-19#Favipiravir-maycombat-SARS-CoV-2. file:///C:/Users/av/ Downloads/Experimental_Treatment_with_Favipiravir_for_COVID-%20(1).pdf. https://www.precisionvaccinations.com/vaccines/avigan-antiviral-medication.];
- at a dose of 1800 mg twice a day on the first day, and then 800 mg twice a day for 14 days [https://bgr.com/2020/04/17/coronavirus-update-antiviral-tablet-avigan-clinical-trial/. https://theprint.in/health/glenmark-enrols-150-patients-to-test-favipiravir-the-most-sought-after-drug-for-covid/420019/.].

It was found that in combination treatment of patients with confirmed COVID-19 who received FVP orally (day 1: 1600 mg twice a day; days 2-14: 600 mg twice a day, 14 days) plus inhalation of interferon-α aerosol (5 million units twice a day), a viral clearance of 4 days (2.5-9) was achieved against 11 days (8-13) for a control group of patients receiving lopinavir/ritanovir (400 mg/100 mg twice a day)
[file:///C:/Users/av/Downloads/Experimental_Treatment_with_Favipiravir_for_COVID-%20(1).pdf].

It is also known that FVP has been used in doses of 30 mg/kg/day in two to four divided doses [https://emedz.net/antiviral-drugs-for-the-treatment-of-covid-19-infection/].

In February 2020, FVP was successfully tested in China in a randomized trial of antiviral therapy for SARS-CoV-2 coronavirus (COVID-19). In February 2020, FVP was approved in China as an effective antiviral agent against COVID-19 [https://de.wikipedia.org/wiki/Favipiravir].

As of May 22, 2020, the number of people infected with COVID-19 worldwide is 5,214,971, of which 335,002 have died and 2,094,920 have recovered. Top 10 countries by number of coronavirus cases: USA (1,621,333), Russia (326,448), Brazil (310,921), Spain (280,117), Great Britain (250,908), Italy (228,006), France (181,826), Germany (179,110), Turkey (153,548), Iran (129,341) https://sport24.ru/news/zozh/2020-05-22-koronavirus-na-22-maya-statistika-v-mireonlayn-rossiya-karta-na-segodnya.

Given that SARS-CoV-2 poses a serious threat to the world's public health and economy, it seems appropriate to search for novel methods of treatment for RNA viral infections.

The subject of this invention is a method for treating RNA viral infections, including COVID-19 (SARS-CoV-2), which consists in administering a pharmaceutical composition in the form of a tablet, capsule, pill, or powder to RNA virus infected patients, said pharmaceutical composition containing FVP as the active ingredient, at a dose of ≥40 mg/kg/day on day 1 and at a dose of ≥16 mg/kg/day on subsequent days until the virus leaves the body, optionally in combination with concomitant medications.

For patients with a body weight less than 75 kg, more preferable doses are: 1600 mg 2 times a day on day 1 and 600 mg 2 times a day on subsequent days until the virus leaves the body.

For patients with a body weight from 75 kg to 90 kg, inclusive, more preferable doses are: 2000 mg 2 times a day on day 1 and 800 mg 2 times a day on subsequent days until the virus leaves the body.

For patients with a body weight over 90 kg, more preferable doses are: 2400 mg 2 times a day on day 1 and 1000 mg 2 times a day on subsequent days until the virus leaves the body.

Depending on the patient's condition, concomitant medications may be administered, including: analgesics, anticoagulants, antibacterial drugs for systemic use, plasma-substituting and perfusion solutions, antitussive drugs and anti-cold agents, beta-blockers, vitamins, diuretics, drugs for diseases associated with impaired acidity, drugs that affect the renin-angiotensin system, drugs for diabetes, calcium channel blockers, corticosteroids for systemic use, drugs for heart diseases, antidiarrhoeal drugs, antiprotozoal drugs, immunosuppressants, drugs for thyroid diseases, drugs for obstructive respiratory diseases, antitumor hormonal drugs, hypolipidemic drugs, other drugs for gastrointestinal diseases and metabolic disorders.

Preferred concomitant medications are analgesics, anticoagulants, antibacterial drugs for systemic use, plasma-substituting and perfusion solutions, antitussive drugs, anti-cold agents, and vitamins.

Clinical studies of the drug Avifavir, which includes FVP as the active ingredient, showed that the median daily dose of FVP on day 1 per 1 kg of body weight in patients with a negative PCR test result for SARS-CoV-2 four days after the start of treatment was 44 mg/kg, and in patients with a positive result, 39 mg/kg. For a dose of FVP ≥43 mg/kg on day 1, the virus elimination rate was 79%, and in patients with a dose of <43 mg/kg, 44%. The median elimination of the virus in patients took 4 days against 9 for patients treated with standard therapy. In 68% of patients who took Avifavir, the body temperature normalized on day 3, while in the control group on day 6. The average viral clearance in Avifavir-treated patients took 4 days, while in the standard therapy group, 9 days. After the first 4 days of treatment, 65% out of 40 patients treated with Avifavir had a negative test result for coronavirus, which is 2 times higher than in the standard therapy group (30%). By day 10, the number of patients with a negative test result reached 35 out of 40.
This invention is illustrated by, but not limited to, the following example.

**Example.** "Adaptive, multicenter, randomized, open-label, comparative clinical study of the efficacy and safety of Avifavir in moderate-severity patients hospitalized with COVID-19."

Study objective: Evaluation of the antiviral effect of Avifavir (tablets containing 200 mg of FVP as the active ingredient [Patent application RU 2020116521 of 07.05.20]) based on the rapidity of SARS-CoV-2 elimination.

The study involved 60 patients with a confirmed new coronavirus infection (COVID-19) of moderate severity who were admitted to infectious departments of clinical centers.

Patients were randomized into 3 study therapy groups. Group 1 (20 patients): the FVP dose was 1600 mg 2 times on day 1, then 600 mg 2 times a day for 13 days. Group 2 (20 patients): the FVP dose was1800 mg 2 times on day 1, then 800 mg 2 times a day for 13 days.

Depending on the condition, the patients of both groups took, along with FVP, concomitant medications, including: analgesics, anticoagulants, antibacterial drugs for systemic use, plasma-substituting and perfusion solutions, antitussive drugs and anti-cold agents, beta-blockers, vitamins, diuretics, drugs for diseases associated with impaired acidity, drugs that affect the renin-angiotensin system, drugs for diabetes, calcium channel blockers, corticosteroids for systemic use, drugs for heart diseases, Antidiarrhoeal drugs, Antiprotozoal drugs, immunosuppressants, drugs for thyroid diseases, drugs for obstructive respiratory diseases, antitumor hormonal drugs, hypolipidemic drugs, other drugs for gastrointestinal diseases and metabolic disorders.

Most often, the patients took, along with FVP, concomitant medications selected from the series of analgesics, anticoagulants, antibacterial drugs for systemic use, plasma-substituting and perfusion solutions, antitussive drugs, anti-cold agents, and vitamins.

The patients of the standard therapy group (20 people) took, depending on a particular patient's condition, Chloroquine, Azithromycin, Amoxiclav, Azithromycin+Hydroxychloroquine, Azithromycin+Ceftriaxone or Azithromycin+Lopinavir+Ritonavir.

At the time of inclusion in the study, 27% of patients required oxygen support and 37% were at risk for severe disease (age over 60 and/or presence of chronic comorbidities). The average duration of the disease from the onset of the first symptoms to the beginning of the study therapy was 7 days. Among the most common symptoms of the disease were fever above 37.5° (95%), cough (83%), weakness (70%), anosmia (35%), chest congestion (30%), and shortness of breath (28%). Initial parameters indicate a slightly lighter course of the disease in the standard therapy group (saturation within the normal range in 95% of patients, 45% of young patients).

Clinical studies of the drug Avifavir, which includes FVP as the active ingredient, showed that the median daily FVP dose on day 1 per 1 kg of body weight in patients with a negative PCR test result for SARS-CoV-2 4 days after the start of treatment was 44 mg/kg, and in patients with a positive test result, 39 mg/kg. For an FVP dose of ≥ 43 mg/kg on day 1, the viral elimination rate was 79%, and in patients who took < 43 mg/kg, 44%. The median viral elimination in patients took 4 days against 9 in the case of standard therapy. In 68% of patients who took Avifavir, the body temperature normalized on day 3, while in the control group, on day 6. The average viral clearance in Avifavir-treated patients took 4 days, while in the standard therapy group, 9 days. After the first 4 days of treatment, 65% out of 40 patients treated with Avifavir had a negative test result for coronavirus, which is 2 times higher than in the standard therapy group (30%). By day 10, the number of patients with a negative test result reached 35 out of 40.

## Claims

1. A method for treating RNA viral infections, including COVID-19 (SARS-CoV-2), which consists in administering to the patient a pharmaceutical composition in the form of a tablet, capsule, pill, or powder containing Favipiravir as the active ingredient at a dose of ≥40 mg/kg/day on day 1 and at a dose of ≥16 mg/kg/day on subsequent days until the virus leaves the body, optionally in combination with concomitant medications.

2. The method according to Claim 1, wherein the dose of Favipiravir administered to a patient with a body weight less than 75 kg is 1600 mg 2 times a day on day 1 and 600 mg 2 times a day on subsequent days.

3. The method according to Claim 1, wherein the dose of Favipiravir administered to a patient with a body weight from 75 kg to 90 kg is 2000 mg 2 times a day on day 1 and 800 mg 2 times a day on subsequent days.

4. The method according to Claim 1, wherein the dose of Favipiravir administered to a patient with a body weight over 90 kg is 2400 mg 2 times a day on day 1 and 1000 mg 2 times a day on subsequent days.

5. The method according to any of Claims 1-4, wherein a concomitant medication is administered, which is selected from the series of analgesics, anticoagulants, antibacterial drugs for systemic use, plasma-substituting and perfusion solutions, antitussive drugs and anti-cold agents, beta-blockers, vitamins, diuretics, drugs for diseases associated with impaired acidity, drugs that affect the renin-angiotensin system, drugs for diabetes, calcium channel blockers, corticosteroids for systemic use, drugs for heart diseases, antidiarrhoeal drugs, antiprotozoal drugs, immunosuppressants, drugs for thyroid diseases, drugs for obstructive respiratory diseases, antitumor hormonal drugs, hypolipidemic drugs, other drugs for gastrointestinal diseases and metabolic disorders.

6. The method according to Claim 5, wherein the concomitant medication is selected from the series of analgesics, anticoagulants, antibacterial drugs for systemic use, plasma-substituting and perfusion solutions, antitussive drugs, anti-cold agents, and vitamins.
